# EUROPEAN PATENT APPLICATION

(11) **EP 4 410 980 A1**
(43) Date of publication of application: **07.08.2024**
(21) Application number: 22876367.8
(22) Date of filing: 28.09.2022
(51) Int. Cl.: C12N 15/12, C12Q 1/686

(54) **METHOD FOR MEASURING CELL CONCENTRATION**

(30) Priority: 29.09.2021 JP 2021160065
(71) Applicant: Takeda Pharmaceutical Company Limited, Osaka-shi, Osaka 541-0045 (JP)
(72) Inventor: YAMAMOTO, Shunsuke, Fujisawa-shi, Kanagawa 251-0012 (JP); NAKAYAMA, Miyu, Fujisawa-shi, Kanagawa 251-0012 (JP); HIRABAYASHI, Hideki, Fujisawa-shi, Kanagawa 251-0012 (JP)
(74) Representative: Jones, Nicholas Andrew
(86) International application number: PCT/JP2022/036238
(87) International publication number: WO 2023/054501

(57) **Abstract**

Disclosed is a method for measuring a cell concentration in a sample, the method including the following steps of: (1) measuring the number of copies of a specific gene and a gene of an external standard in a plurality of standard samples each having a known cell concentration by using digital PCR (dPCR); (2) normalizing the measured value of the specific gene measured in the step (1) using the measured value of the external standard and creating a calibration curve on the basis of the normalized value and the known cell concentration; (3) measuring the number of copies of the same specific gene and gene of the external standard in a sample having an unknown cell concentration by using dPCR; and (4) normalizing the measured value of the specific gene measured in the step (3) using the measured value of the external standard and determining a cell concentration using the calibration curve created in the step (2) from the normalized value.

## Description

### Technical Field

The present invention relates to a method for measuring a cell concentration in a sample.

### Background of Invention

As part of the treatment of various diseases, research and development of cell therapy, in which desired cells or tissue are prepared by induction of differentiation from human-derived stem cells (for example, iPS cells (induced pluripotent stem cells) and ES cells (embryonic stem cells)), then transplanted to a patient, have been advanced. In cell therapy products, measurement of cell concentration is necessary, but methods currently used are complicated, and there is no established method as a simple and efficient measurement method.

NPL 1 reports a method of quantifying a cell concentration using a calibration curve created by quantitative PCR (qPCR), and it is stated that a calibration curve is required for each tissue. It is described that a target sequence of PCR is Alu gene.

NPL 2 reports that quantitative PCR (qPCR) performed using primers/probes designed based on the LINE-1 sequence enables quantification of human cells in various animals including non-human primates. The same is reported in PTL 1, and it is described that, by using a probe capable of hybridizing to a specific sequence of the human LINE-1 gene, human cells in a sample collected from an animal other than a human to which human cells are transplanted or administered can be quantified and detected by qPCR.

NPL 3 discloses a method for measuring the number of copies of a target gene (CAR transgene) by qPCR using canine genomic DNA as an external standard gene in order to analyze cellular kinetics of chimeric antigen receptor (CAR) T cells in a biological sample. A report similar to this is also made in PTL 2.

Real-time PCR is mainly used as qPCR used in the above literatures. As qPCR, digital PCR (dPCR) is also known. dPCR is performed by performing PCR after limiting dilution of a biological sample so that 0 or 1 (or a plurality of) target gene(s) is/are contained in a microcompartment, and estimating the absolute number of copies of the target gene in the biological sample from the ratio of the number of microcompartments in which the amplification signal becomes negative (not containing the target gene) to the total number of microcompartments.

### Citation List

### Patent Literatures

PTL 1: WO2019/240073A
PTL 2: WO2021/065877A

### Non-patent Literatures

NPL 1: Shimizu et al., Regenerative Therapy 15 251-257, 2020
NPL 2: Yamamoto et al., Drug Metabolism and Pharmacokinetics 36 100359, 2021
NPL 3: Yamamoto et al., Scientific Reports volume 10 17884, 2020

### Summary of Invention

### Technical Problem

The matrix is a substance (for example, a substance that is extracted at the same time when DNA is extracted from a biological sample and cannot be removed) other than an object to be measured contained in a measurement sample. In the case of quantifying human cells in a sample collected from an animal including a human to which human cells are transplanted or administered by conventional real-time PCR, since the matrix has a different composition for each biological sample (organ and blood), it is necessary to create a calibration curve for each type of biological sample because the quantification is affected by the type of biological sample to be collected (matrix effect).

In the case of using an external standard for normalizing a DNA recovery rate when DNA is extracted from a cell, it is necessary to separately measure a target gene and a gene of the external standard in real-time PCR.

An object of the present invention is to provide a method for measuring a cell concentration in a sample, which enables simultaneous measurement of a target gene and a gene of an external standard and enables quantification of the number of cells with high accuracy even when calibration curves of different matrices are used.

### Solution to Problem

The present inventors have conducted intensive studies to achieve the above object, and as a result, have found that it is possible to quantify the number of cells with high accuracy even when calibration curves of different matrices are used, by measuring the number of copies of a target gene and a gene of an external standard in a standard sample by using dPCR, creating a calibration curve using the measured value normalized with the external standard, and then determining the cell concentration using the calibration curve using a value obtained by normalizing the number of copies of the target gene in the sample measured by using dPCR with the external standard. By using dPCR in this way, it is possible to simultaneously measure a target gene and a gene of an external standard.

The present invention has been completed by further conducting studies based on these findings, and provides a method for measuring a cell concentration in a sample described below.

[1] A method for measuring a cell concentration in a sample, the method including the following steps of:
   (1) measuring the number of copies of a specific gene and a gene of an external standard in a plurality of standard samples each having a known cell concentration by using digital PCR (dPCR);
   (2) normalizing the measured value of the specific gene measured in the step (1) using the measured value of the external standard and creating a calibration curve on the basis of the normalized value and the known cell concentration;
   (3) measuring the number of copies of the same specific gene and gene of the external standard in a sample having an unknown cell concentration by using dPCR; and
   (4) normalizing the measured value of the specific gene measured in the step (3) using the measured value of the external standard and determining a cell concentration using the calibration curve created in the step (2) from the normalized value.
[1a] The method according to [1], wherein the digital PCR is droplet digital PCR (ddPCR).
[2] The method according to [1] or [1a], wherein in the step (1), measurement of the number of copies of a specific gene and measurement of the number of copies of a gene of an external standard are simultaneously performed.
[3] The method according to [1] or [2], wherein a matrix of each of the standard samples used for creating a calibration curve is different from a matrix of the sample used for measuring a cell concentration.
[4] The method according to any one of [1] to [3], wherein accuracy of the value of the cell concentration determined in the step (4) is within ±35%.
[5] The method according to any one of [1] to [4], wherein precision of the value of the cell concentration determined in the step (4) is within ±35%.
[6] The method according to any one of [1] to [5], wherein the specific gene is LINE-1 gene.
[7] The method according to any one of [1] to [5], wherein the specific gene is REXO1L1 gene.

### Advantageous Effects of Invention

According to the method for measuring a cell concentration of the present invention, it is possible to quantify the number of cells with high accuracy even when calibration curves of different matrices are used. It is possible to simultaneously measure a target gene and a gene of an external standard.

### Brief Description of Drawings

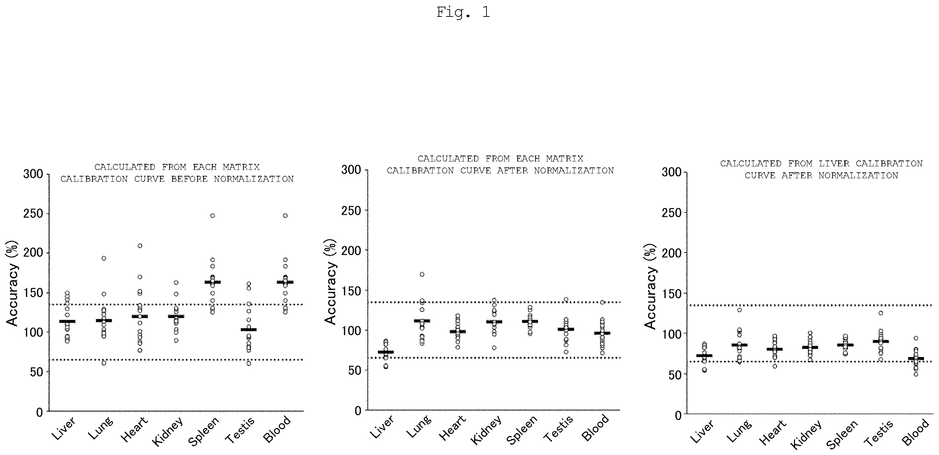

Fig. 1 is a graph showing a measurement result by qPCR in Test Example 1. Left: accuracy (%) calculated from each matrix calibration curve before normalization, middle: accuracy (%) calculated from each matrix calibration curve after normalization, right: accuracy (%) calculated from liver calibration curve after normalization.

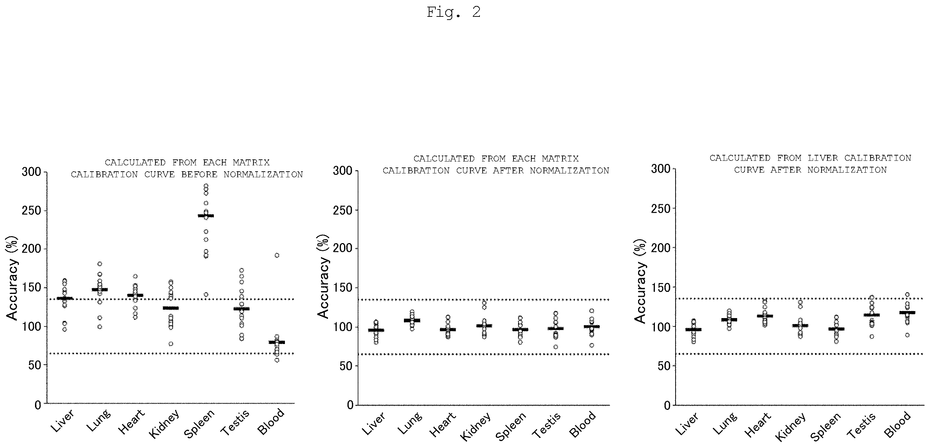

Fig. 2 is a graph showing a measurement result by ddPCR in Test Example 1. Left: accuracy (%) calculated from each matrix calibration curve before normalization, middle: accuracy (%) calculated from each matrix calibration curve after normalization, right: accuracy (%) calculated from liver calibration curve after normalization.

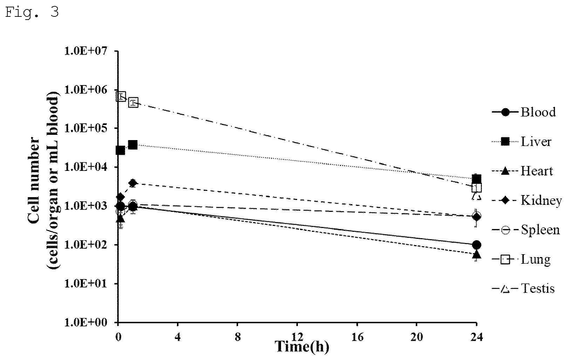

Fig. 3 is a graph showing the number of human cells in each tissue and blood sample of a mouse intravenously administered with human cells in Test Example 4.

### Description of Embodiments

Hereinafter, embodiments of the present invention will be described in detail.

The term "comprise(s)" or "comprising" means inclusion of the element(s) following the word without limitations thereto. Accordingly, this suggests inclusion of the element(s) following the word, but does not suggest exclusion of any other element. The phrase "consist(s) of" of "consisting of" means inclusion of all the element(s) following the phrase and limitation thereto. Accordingly, the phrase "consist(s) of" or "consisting of" indicates that the enumerated element(s) is required or essential and substantially no other elements exist. The phrase "consist(s) essentially of" or "consisting essentially of" means inclusion of any element following the phrase and limitation of other elements to those that do not affect the activity or effect of the enumerated element(s) specified in the present disclosure. Accordingly, the phrase "consist(s) essentially of" or "consisting essentially of" indicates that the enumerated element(s) is required or essential, but other elements are optional and may exist or not exist depending on whether they affect the activity or effect of the enumerated element(s).

The "accuracy" used herein is the degree of accuracy indicating that the value is close to the true value. That is, the "accuracy" becomes higher as the mean value is closer to the true value.

The "precision" used herein is a scale indicating that there is a little variation among the values obtained in multiple measurements.

A method for measuring a cell concentration in a sample of the present invention includes the following steps of:
(1) measuring the number of copies of a specific gene and a gene of an external standard in a plurality of standard samples each having a known cell concentration by using digital PCR (dPCR);
(2) normalizing the measured value of the specific gene measured in the step (1) using the measured value of the external standard and creating a calibration curve on the basis of the normalized value and the known cell concentration;
(3) measuring the number of copies of the same specific gene and gene of the external standard in a sample having an unknown cell concentration by using dPCR; and
(4) normalizing the measured value of the specific gene measured in the step (3) using the measured value of the external standard and determining a cell concentration using the calibration curve created in the step (2) from the normalized value.

In the present invention, the sample for measuring a cell concentration is not particularly limited, examples thereof include a biological sample collected from a non-human animal to which human cells have been transplanted or administered and a biological sample collected from a human to which human cells have been transplanted or administered, and a biological sample collected from a non-human animal to which human cells have been transplanted or administered is more preferable.

The "non-human animal" refers to all non-human animals, typically experimental animals (including animal disease models) to which human cells are transplanted or administered. Examples of such experimental animals include non-human primates (such as marmosets, cynomolgus monkeys, rhesus monkeys, tufted capuchin, and chimpanzees), cows, pigs, goats, sheep, dogs, rabbits, and rodents (such as mice and rats). Examples of non-human primates (Primates) include chimpanzees (Panina), gorillas (Gorillini), orangutans (Ponginae), gibbons (Hylobatidae), guenons (Cercopithecoidea and Cercopithecidae), spider monkeys (Atelidae), tarsiers (Tarsiidae), and lemurs (Lemuriformes and Lemuridae). Examples of guenons (monkeys of the family Cercopithecidae) include monkeys of the genus Macaca such as cynomolgus monkey, rhesus monkeys, and Japanese macaques.

The biological sample to be collected from a non-human animal or a human is not particularly limited as long as a sample for dPCR can be prepared, and various organs, tissues, cell populations, and body fluids can be used as biological samples. Examples of the organs from which biological samples can be collected include brain (e.g.: sites such as olfactory bulb, amygdala, basal ganglia, hippocampus, thalamus, hypothalamus, hypothalamic nucleus, cerebral cortex, medulla oblongata, cerebellum, occipital lobe, frontal lobe, temporal lobe, putamen, caudate nucleus, corpus callosum, and substantia nigra, the whole brain, and the like), spinal cord, pituitary gland, stomach, pancreas, kidney, liver, genital gland, thyroid gland, gallbladder, bone marrow, adrenal gland, skin, muscles, lungs, gastrointestinal tract (e.g.: large intestine, small intestine, and the like), blood vessels, heart, thymus, spleen, submandibular glands, peripheral blood, peripheral blood cells, prostate, testicles, testis, ovaries, placenta, uterus, bones, joints, and skeletal muscles, and tissues and cell populations contained in those organs can also be used as biological samples. The biological sample may be a cancerous tissue or cell population. Examples of the body fluids include blood, plasma, serum, lymph, cerebrospinal fluid, saliva, bile, urine, and feces.

The human cells to be transplanted or administered to a non-human animal or a human are not particularly limited as long as they can be transplanted or administered to the organs of non-human animals as described above or a human, and human genomic DNA can be extracted when a sample for dPCR is prepared. Examples of such human cells include human-derived spleen cells, nerve cells, glial cells, pancreatic β cells, bone marrow cells, mesangial cells, Langerhans cells, epidermal cells, epithelial cells, endothelial cells, fibroblasts, fiber cells, muscle cells (e.g.: skeletal myocytes, cardiomyocytes, myoblasts, and myosatellite cells), adipocytes, immune cells (e.g.: macrophages, T cells, B cells, natural killer cells, mast cells, neutrophils, basophils, eosinophils, monocytes, and megakaryocytes), synoviocytes, chondrocytes, osteocytes, osteoblasts, osteoclasts, mammary gland cells, hepatocytes, stromal cells, egg cells, and sperm cells, as well as the stem cells (including induced pluripotent stem cells (iPS cells) and embryonic stem cells (ES cells)), progenitor cells, blood cells, oocytes, and fertilized eggs that can be induced to differentiate into these cells. The human cells also include the human-derived cells prepared by inducing differentiation from the above stem cells and the like in vitro.

Before the measurement by dPCR, a sample can be appropriately subjected to various pretreatments. Examples of such pretreatments include an extraction treatment of DNA from a biological sample. A method for extracting DNA is not particularly limited, a known method can be appropriately selected, and examples thereof include a phenol/chloroform extraction method, a cetyltrimethylammonium bromide (CTAB) method, an alkaline thermal extraction method, a commercially available DNA extraction kit (e.g.: MagMAX DNA Multi-Sample Ultra 2.0 Kit), an extraction method using a commercially available DNA extraction instrument (e.g.: KingFisher Flex).

The specific gene in the present invention is a gene used for measuring a cell concentration, and can be selected from, for example, genes contained in human cells. In particular, a gene capable of quantifying human cells in various animals including non-human primates, that is, a gene having a sequence specific for human is preferable. Examples of such a specific gene include LINE-1, REXO1L1, Alu, DUF1220, SPDYE3, GOLGA8G, HRNR, USP17, GOLGA6L9, PSG3, and TCEB3C. When human cells are transplanted or administered to a human, if there is a different gene sequence between the cell to be transplanted or administered and the human to which the cells are transplanted or administered, the human cells can be quantified by targeting the site thereof. For example, when cells to be transplanted or administered are male cells and a human to which the cells are transplanted or administered is a female, targeting a Y chromosome specific gene, utilizing SNP, and the like are mentioned. In the present invention, the specific gene is not limited to a region encoding a protein and RNA.

The nucleotide sequence of the human LINE-1 ("Long interspersed nucleotide factor-1", "Long interspersed nuclear elements-1") gene is registered in GenBank database provided by NCBI (National Center for Biotechnology Information) according to a predetermined accession number (Accession No.) (for example, GenBank Accession No.: KP237032, GenBank Accession No.: JN698885, GenBank Accession No.: GU477637, and the like). Although there are many families of human LINE-1 genes, the "human LINE-1 gene" used herein also includes family genes other than those exemplified above.

The nucleotide sequence of the human REXO1L1 ("REXO1 like 1", REXO1L1P, GOR) gene (Entrez Gene ID: 254958) is registered in RefSeq database provided by NCBI according to a predetermined accession number (Accession No.) (RefSeq Accession No.: NG_053157 REGION: 101..1255).

The human Alu (Arthrobacter luteus) gene is originally a short chain of DNA characterized by the action of Alu restriction enzyme, and is the most abundant transportable element present dispersed in 1 million or more copies in the human genome, thus making it possible to detect human cell-derived genomic DNA in animals with high sensitivity (see NPL 1).

The human DUF1220 gene (Oldubai protein domain) (Entrez Gene ID: 400818) has the largest number of copies specifically for human among all coding regions on the genome, and is a multiple copy gene similar to the Alu gene specific for human.

Gene IDs of genes other than the above genes are shown below.
Human SPDYE3 ("speedy/RINGO cell cycle regulator family member E3", SPDYB2) gene: Entrez Gene ID: 441272
Human GOLGA8G ("golgin A8 family member G", GOLGA8F) gene: Entrez Gene ID: 283768
Human HRNR ("hornerin", FLG3, S100A16, S100a18) gene: Entrez Gene ID: 388697
Human USP17 ("ubiquitin specific peptidase 17 like family member 2", USP17L2, DUB3, DUB-3) gene: Entrez Gene ID: 377630
Human GOLGA6L9 ("golgin A6 family like 9", GOLGA6L20) gene: Entrez Gene ID: 440295
Human PSG3 ("pregnancy specific beta-1-glycoprotein 3") gene: Entrez Gene ID: 5671
Human TCEB3C ("elongin A3, pseudogene", ELOA3P, ELOA3, ELOA3A, HsT829, ELOA3AP, TCEB3L2) gene: Entrez Gene ID: 162699

The gene of the external standard in the present invention is a gene used as an external standard for normalizing a quantitative value of a specific gene in dPCR, and is a DNA having a nucleotide sequence that does not intersect with the specific gene, that is, is not amplified by a primer for the specific gene. Conversely, the gene of the external standard needs to be a nucleotide sequence that does not intersect with the specific gene and a gene in another biological sample. The gene of the external standard desirably includes a nucleotide sequence (gene or the like) not possessed by a human. By using such a gene of the external standard, it is possible to suppress the influence of the variation in the recovery rate of the specific gene on the quantitative value, and accuracy can be improved.

The gene of the external standard is not particularly limited as long as it is a gene having a nucleotide sequence having the characteristics as described above. Examples of typical genes of an external standard include genomic DNA of mammals other than humans, preferably mammals other than primates, such as cows, pigs, goats, sheep, dogs, cats, rabbits, rodents (such as mice and rats). As the gene of the external standard, an artificial nucleic acid containing an artificially designed and synthesized sequence may be used. The gene of the external standard is not limited to a region encoding a protein and RNA, and the entire region of genomic DNA can be widely used.

The gene of the external standard may be a DNA isolated in advance so that the addition amount can be easily adjusted, or may be a DNA in a state of being contained in a cell of a mammal other than a human. The amount of the gene of the external standard added to the sample is not particularly limited, and can be appropriately adjusted.

dPCR is performed by performing PCR after limiting dilution of a biological sample so that 0 or 1 (or a plurality of) target gene(s) is/are contained in a microcompartment (well or droplet), and estimating the absolute number of copies of the target gene in the biological sample from the ratio of the number of microcompartments in which the amplification signal becomes negative (not containing the target gene) to the total number of microcompartments. At the time of this estimation, the possibility of existence of a microcompartment containing a plurality of target genes is normalized using the Poisson distribution. The amplification signal in dPCR can be based on a fluorescence probe method (for example, a TaqMan method, a molecular beacon method, or a cycling probe method), an intercalator method using a reagent that emits fluorescence by binding to double-stranded DNA, or the like. In the present invention, dPCR is preferably droplet digital PCR (ddPCR), and ddPCR is dPCR in which droplets are produced by using a microchannel technology or the like when a microcompartment is produced, and PCR is performed in each droplet.

Basic reagents, kits, apparatuses, and the like for carrying out dPCR are also commercially available, and these can also be used in the present invention. Examples of commercially available products of such apparatuses of dPCR include QX200 Droplet Digital PCR System, and QX ONE Droplet Digital PCR System manufactured by BIO-RAD, QuantStudio (trademark) 3D digital PCR System manufactured by Thermo Fisher Scientific Inc., and QIAcuity Digital PCR System manufactured by QIAGEN. Those skilled in the art can appropriately design the base sequence and the base length of each primer (forward primer and reverse primer) and each probe used in the case of adopting the fluorescence probe method for each of a specific gene and a gene of an external standard necessary for performing dPCR so that each of the specific gene and the gene of the external standard can be specifically amplified (so as not to intersect with other genes) (see, for example, WO2019/240073A). The probe and the primer may be a sequence completely complementary to the target sequence, a sequence partially including a mismatch, or a base sequence having a homology in a range capable of hybridizing with these sequences at high stringency (stringent hybridization conditions). The fluorescent substance used in the case of adopting the fluorescence probe method and the quencher forming a FRET pair with the fluorescent substance are not particularly limited, and can be appropriately determined by those skilled in the art. The number of copies of a gene in dPCR can be measured according to a known method, and for example, can be measured according to a manual of a manufacturer of a dPCR apparatus.

### - Step (1)

In the step (1), in order to create a calibration curve, the number of copies of a specific gene and a gene of an external standard in a plurality of standard samples each having a known cell concentration is measured by using dPCR.

As a method for preparing a standard sample, a method capable of adjusting the concentration of cells can be widely used. Examples thereof include a method of visually counting the number of cells with a microscope using a hemocytometer, a method using an automatic cell counter, and a method using a flow cytometer. If one type of cell concentration can be adjusted, the other cell concentrations can be adjusted by stepwise dilution. A standard sample can be prepared by adding cells of each concentration thus adjusted to the biological sample. The cells may be added to a culture medium for cell culture and a buffer other than the biological sample.

The number of concentrations of the standard sample used in the step (1) is not particularly limited as long as a calibration curve can be made, and is, for example, 2 or more, preferably 3 or more, more preferably 4 or more, still more preferably 5 or more, and particularly preferably 6 or more, and the upper limit is, for example, 30.

The number of standard samples for each known concentration used in the step (1) is not particularly limited, and is, for example, 1 or more, preferably 2 or more, and more preferably 2.

In the step (1), in the case of using a standard sample having a known cell concentration as quality control (QC) instead of creating a calibration curve, the number of standard samples for each known concentration is not particularly limited, and is, for example, 3 or more, preferably 4 or more, more preferably 5 or more, and still more preferably 6 or more, and the upper limit is, for example, 100.

By performing dPCR, since measurement of the number of copies of a specific gene and measurement of the number of copies of a gene of an external standard can be simultaneously performed, it is preferable to simultaneously perform measurement of the number of copies of a specific gene and measurement of the number of copies of a gene of an external standard.

### - Step (2)

In the step (2), the measured value of the specific gene measured in the step (1) is normalized using the measured value of the external standard and a calibration curve is created on the basis of the normalized value and the known cell concentration.

A method of normalizing the measured value of the specific gene measured in the step (1) using the measured value of the external standard is not particularly limited, and for example, (the measured value of the specific gene/the measured value of the external standard) is determined, and this determined value can be used as a normalized value.

The calibration curve can be created using software or the like by a conventional method such as a least squares method.

### - Step (3)

In the step (3), the number of copies of the same specific gene and gene of the external standard in a sample having an unknown cell concentration is measured by using dPCR.

By performing dPCR, since measurement of the number of copies of a specific gene and measurement of the number of copies of a gene of an external standard can be simultaneously performed, it is preferable to simultaneously perform measurement of the number of copies of a specific gene and measurement of the number of copies of a gene of an external standard.

### - Step (4)

In the step (4), the measured value of the specific gene measured in the step (3) is normalized using the measured value of the external standard and a cell concentration is determined using the calibration curve created in the step (2) from the normalized value.

A method of normalizing the measured value of the specific gene measured in the step (3) using the measured value of the external standard is not particularly limited, and for example, (the measured value of the specific gene/the measured value of the external standard) is determined, and this determined value can be used as a normalized value. The final measured value is the number of cells per unit amount (mass, capacity) of the sample, and can be expressed, for example, in units such as "cells/µL blood" and "cells/mg tissue".

The accuracy of the value of the cell concentration determined in the step (4) is preferably within ±35%, more preferably within ±30%, still more preferably within ±25%, particularly preferably within ±20%, and most preferably within ±15%. The value of "accuracy" here is a difference between the mean of the determined cell concentration values and the known concentration expressed as a percentage relative to the known concentration (relative error: RE).

It is also desirable that the accuracy of the value of the cell concentration determined in the step (4) satisfies the following criteria:
(1) 67% or more of all QC samples are within the specified value,
(2) 50% or more at each concentration are within the specified value, and
(3) a mean value of each concentration is within the specified value.

Examples of the specified value include ±35%, ±30%, ±25%, ±20%, and ±15%.

The precision of the value of the cell concentration determined in the step (4) is preferably within ±35%, more preferably within ±30%, still more preferably within ±25%, particularly preferably within ±20%, and most preferably within ±15%. The value of "precision" here means a coefficient of variation (CV).

The accuracy and precision described above can be calculated by using a standard sample having a known cell concentration as QC.

By measuring a cell concentration in a sample by the method of the present invention, even when a matrix of a standard sample used for creating a calibration curve is different from a matrix of a sample used for measuring a cell concentration, it is possible to quantify the number of cells with high accuracy. Therefore, in order to efficiently measure a cell concentration, it is desirable that a matrix of a standard sample used for creating a calibration curve is different from a matrix of a sample used for measuring a cell concentration. That is, a calibration curve is created using one type of matrix, and the number of cells of other various types of matrices can be quantified using the calibration curve.

### Examples

Examples will be given in the following to describe the present invention in more detail. However, the present invention is not limited to these Examples at all. Note that qPCR in Examples means real-time PCR.

### - Primers and probes

In the following experiments, the following primers and probes were used.

### - LINE1

SEQ ID NO: 1: TGAGTTCATATCCTTTGTAGGGA
SEQ ID NO: 2: CCATTACTGGGTATATACCCAAATGAG
SEQ ID NO: 3: GCGCTGCACCCACTAATGT

### - External standard gene

SEQ ID NO: 4: GCCTTGGCTGCGCAGGCTGCTGTGGTGCAG
SEQ ID NO: 5: CGCCCATGTATTACTTCATCTGTTGCC
SEQ ID NO: 6: CACGGCGATGGCGCCCAGGAA

### - REXO1L1

SEQ ID NO: 7: TTGCCCCAAGTCCAA
SEQ ID NO: 8: TCGCCAAGACGAGCATCA
SEQ ID NO: 9: TTTGGGCGCTGAAAAAGCT

### Test Example 1 Amplification of human cell-derived DNA in C.B-17 SCID mouse sample

An ATL solution (150 µE, DNeasy Blood & Tissue Kits, QIAGEN) in which 15 mg of liver, kidney, heart, testis and 7.5 mg of lung and spleen of C.B-17 SCID mouse were dissolved and 50 µL of blood were used as specimens, and calibration curve samples (n = 2) obtained by adding, to these specimens, ATL solutions (70 µE) in which human cells (peripheral blood T cells, Cryo-T8: Human CD8⁺ Negatively Selected (5-8M cells), Precision Bioservices) corresponding to 30, 100, 1,000, 5,000, 30,000, and 100,000 cells were dissolved and a blank sample obtained by adding an ATL solution (70 µL) to which human cells were not added were prepared. QC samples (n = 4) corresponding to 30, 100, 1,000, and 10,000 cells were prepared by the same method as described above. For DNA recovery rate normalization, Dog Genomic DNA, Male (Zyagen) was added as an external standard gene to all samples. Genomic DNA was extracted from each of these samples using a commercially available DNA extraction reagent (DNeasy Blood & Tissue Kits, QIAGEN), and then qPCR quantification and ddPCR quantification were performed on the same extract.

qPCR was quantified using TaqPath ProAmp Master Mixes (including DNA polymerase with 5' → 3' exonuclease activity (Thermo Fisher Scientific Inc.)), the probe of SEQ ID NO: 1 (10 pmol/well), the forward primer of SEQ ID NO: 2 (20 pmol/well), and the reverse primer of SEQ ID NO: 3 (20 pmol/well). qPCR was performed using QuantStudio 7 System (Thermo Fisher Scientific Inc.) under the conditions of 50°C for 2 minutes/95°C for 10 minutes/(95°C for 15 seconds and 60°C for 1 minute), which was regarded as one cycle, and qPCR was performed for 40 cycles. In qPCR, measurement of a target gene and measurement of an external standard gene were separately performed.

ddPCR was quantified by duplex measurement using ddPCR Multiplex Supermix (BIO-RAD), DTT (BIO-RAD), restriction enzyme ScaI (Takara Bio Inc.), the probe of SEQ ID NO: 1 (5 pmol/well), the forward primer of SEQ ID NO: 2 (54 pmol/well) and the reverse primer of SEQ ID NO: 3 (54 pmol/well), the probe of SEQ ID NO: 4 (5 pmol/well), the forward primer of SEQ ID NO: 5 (10 pmol/well), and the reverse primer of SEQ ID NO: 6 (10 pmol/well). ddPCR was performed using QX200 Droplet Digital PCR System (BIO-RAD) at 95°C for 10 minutes/94°C for 30 seconds/(94°C for 30 seconds and 58°C for 1.5 minutes), which was regarded as one cycle, and DNA was amplified at 45 cycles/98°C for 10 minutes.

In the qPCR measurement, a calibration curve was created from the Ct value of the target gene and the number of cells of each calibration curve sample. A calibration curve was created from a difference (Delta Ct) between the Ct value of the target gene and the Ct value of the external standard gene and the number of cells of each calibration curve sample. The accuracy when the number of cells of each QC sample is calculated using these calibration curves is shown in Fig. 1. A circle indicates accuracy of each QC sample, the horizontal line indicates a mean value, and the dotted line indicates ±35%. The accuracy (relative error, RE) of the QC sample calculated using the calibration curve created from the Ct value of the target gene and the number of cells of each calibration curve sample was found to be out of the range of ±35% in some cases, but it was found that the accuracy was improved by normalization with the external standard gene. The results when the accuracy of the QC samples of all organs and blood was calculated from the liver calibration curve normalized with the external standard gene are shown in Fig. 1 and Table 1. Although the accuracy (RE) was generally within ±35% in all organs, in the QC sample of blood, the accuracy was out of the range of ±35% in 38% of the samples (Fig. 1), and in the blood QC sample (1000 cells/50 µL) and the liver QC sample (1000 and 10000 cells/15 mg), the mean value exceeded 35% (Table 1).

In the ddPCR measurement, a calibration curve was created from the number of copies of the target gene and the number of cells of each calibration curve sample. A calibration curve was created from a value obtained by dividing the number of copies of the target gene by the number of copies of the external standard gene and the number of cells of each calibration curve sample. The accuracy when the number of cells of each QC sample is calculated using these calibration curves is shown in Fig. 2. Similar to the results of the qPCR measurement, an improvement in accuracy was observed by normalization with the external standard gene. The results of calculating the accuracy of the QC samples of other matrices from the normalized liver calibration curve are shown in Fig. 2 and Table 1. Although the accuracy (RE) exceeded 35% for each of two samples of testis and blood, the mean value and the accuracy (RE) and precision (coefficient of variation, CV, %) of the mean value at each concentration were within ±35%, which were favorable results. It is considered that excellent quantitativity was obtained by recovery rate normalization with an external standard gene and avoidance of the matrix effect by ddPCR.

**[Table 1]**

| | | qPCR | | | ddPCR | | |
|---|---|---|---|---|---|---|---|
| | Nominal concentration (cells/50 µL blood or 15mg tissue or 7.5mg tissue) | Mean obserbed concentration (cells/50 µL blood or 15mg tissue or 7.5mg tissue) | Relative error (%) | Coefficient of variation (%) | Mean obserbed concentration (cells/50 µL blood or 15mg tissue or 7.5mg tissue) | Relative error (%) | Coefficient of variation (%) |
| Blood | 10,000 | 6,987 | -30.1 | 10.9 | 12,197 | 22.0 | 10.9 |
| | 1,000 | 605 | -39.5 | 7.6 | 1,102 | 10.2 | 4.6 |
| | 100 | 75 | -24.9 | 5.8 | 121 | 21.0 | 8.4 |
| | 30 | 21 | -31.4 | 28.8 | 34 | 14.5 | 18.4 |
| Liver | 10,000 | 6,165 | -38.4 | 13.7 | 9,015 | -9.9 | 7.5 |
| | 1,000 | 649 | -35.1 | 11.0 | 973 | -2.7 | 6.6 |
| | 100 | 79 | -20.9 | 9.5 | 99 | -1.4 | 8.5 |
| | 30 | 25 | -17.0 | 1.6 | 29 | -2.8 | 11.7 |
| Heart | 10,000 | 7,860 | -21.4 | 18.3 | 11,606 | 16.1 | 9.1 |
| | 1,000 | 729 | -27.1 | 6.7 | 1,046 | 4.6 | 3.0 |
| | 100 | 80 | -19.9 | 7.4 | 117 | 17.0 | 9.1 |
| | 30 | 27 | -9.8 | 6.3 | 34 | 12.8 | 8.7 |
| Kidney | 10,000 | 7,676 | -23.2 | 2.9 | 9,601 | -4.0 | 6.8 |
| | 1,000 | 848 | -15.2 | 9.0 | 926 | -7.4 | 7.4 |
| | 100 | 84 | -16.1 | 10.0 | 99 | -0.9 | 5.2 |
| | 30 | 25 | -15.2 | 18.7 | 35 | 16.8 | 11.1 |
| Spleen | 10,000 | 8,730 | -12.7 | 6.0 | 9,554 | -4.5 | 1.4 |
| | 1,000 | 793 | -20.7 | 6.7 | 971 | -2.9 | 3.9 |
| | 100 | 86 | -14.3 | 4.5 | 98 | -1.9 | 11.1 |
| | 30 | 27 | -10.3 | 7.6 | 29 | -3.8 | 15.4 |
| Lung | 10,000 | 8,254 | -17.5 | 16.7 | 10,529 | 5.3 | 8.0 |
| | 1,000 | 754 | -24.6 | 9.3 | 1,046 | 4.6 | 3.2 |
| | 100 | 79 | -21.2 | 20.9 | 108 | 8.2 | 4.6 |
| | 30 | 31 | 4.7 | 18.4 | 35 | 15.4 | 2.7 |
| Testis | 10,000 | 8,923 | -10.8 | 6.5 | 11,759 | 17.6 | 15.5 |
| | 1,000 | 890 | -11.0 | 10.6 | 1,095 | 9.5 | 8.7 |
| | 100 | 104 | 4.4 | 13.7 | 125 | 25.1 | 6.5 |
| | 30 | 23 | -23.6 | 9.0 | 31 | 4.8 | 14.3 |

### Test Example 2 Inter-day repeatability test/amplification of human cell-derived DNA in C.B-17 SCID mouse sample

A DNA/RNA shield solution (200 µL, ZYMO RESEARCH) in which 20 mg of liver of C.B-17 SCID mouse was dissolved was used as a specimen, and calibration curve samples (n = 2) obtained by adding, to the specimen, DNA/RNA shield solutions (70 µE) in which human cells (peripheral blood T cells, Cryo-T8: Human CD8⁺ Negatively Selected (5-8M cells), Precision Bioservices) corresponding to 3, 100, 1,000, 3,000, 5,000, and 10,000 cells were dissolved and a blank sample obtained by adding a DNA/RNA shield solution (70 µL) to which human cells were not added were prepared. QC samples (n = 4) corresponding to 3, 100, 1,000, and 10,000 cells were prepared by the same method as described above using the liver, kidney, heart, lung, spleen, testis, and blood of C.B-17 SCID mouse. Dog Genomic DNA, Male (Zyagen) was added as an external standard gene for DNA recovery rate normalization to all samples. Genomic DNA was extracted from each of these samples using a commercially available DNA extraction reagent (MagMAX DNA Multi-Sample Ultra 2.0 Kit, Applied Biosystems) and Kingfisher Flex (Thermo Fisher Scientific Inc.) as an extraction instrument, and then ddPCR quantification was performed.

ddPCR was quantified by duplex measurement using ddPCR Multiplex Supermix (BIO-RAD), DTT (BIO-RAD), restriction enzyme ScaI (Takara Bio Inc.), the probe of SEQ ID NO: 1 (5 pmol/well), the forward primer of SEQ ID NO: 2 (54 pmol/well) and the reverse primer of SEQ ID NO: 3 (54 pmol/well), the probe of SEQ ID NO: 4 (5 pmol/well), the forward primer of SEQ ID NO: 5 (10 pmol/well), and the reverse primer of SEQ ID NO: 6 (10 pmol/well). ddPCR was performed using QX200 Droplet Digital PCR System (BIO-RAD) at 95°C for 10 minutes/94°C for 30 seconds/(94°C for 30 seconds and 58°C for 1.5 minutes), which was regarded as one cycle, DNA was amplified at 45 cycles/98°C for 10 minutes, and quantification was performed. In order to confirm the inter-day repeatability, the above steps were performed three times.

A calibration curve was created from a value obtained by dividing the number of copies of the target gene by the number of copies of the external standard gene and the number of cells of each calibration curve sample. The results of calculating the number of cells of each QC sample using this calibration curve are shown in Table 2. In any of the QC samples, the mean value of the accuracy (RE) of the values calculated with respect to the number of added cells (Nominal), the accuracy (RE) of the QC sample of 50% or more at each concentration, and the accuracy (RE) and the precision (CV) of the QC sample of 2/3 or more of the total were within ±35%, which were favorable, and excellent quantitativity of this method was shown.

**[Table 2-1]**

| | | 1^{st} assay | | | | | | 2^{nd} assay | | | | | | 3^{rd} assay | | | | | |
|---|---|---|---|---|---|---|---|---|---|---|---|---|---|---|---|---|---|---|---|
| | | Relative error(%) | | | | | | Relative error(%) | | | | | | Relative error(%) | | | | | |
| | Nominal concentration (cells/50 µL blood or 20mg tissue or 10mg tissue) | No.1 | No.2 | No.3 | No.4 | Mean | Coefficient of variation (%) | No.1 | No.2 | No.3 | No.4 | Mean | Coefficient of variation (%) | No.1 | No.2 | No.3 | No.4 | Mean | Coefficient of variation (%) |
| Blood | 10,000 | -10.6 | 15.8 | 14.2 | -10.9 | 2.1 | 14.6 | -15.0 | -5.8 | -7.8 | -19.6 | -12.0 | 7.3 | -22.6 | -3.1 | -1.7 | -20.3 | -11.9 | 12.5 |
| | 1,000 | -18.0 | -16.7 | -14.9 | -18.8 | -17.1 | 2.1 | -13.6 | -12.1 | -17.8 | -16.2 | -14.9 | 3.0 | -2.1 | -2.9 | -1.8 | -6.3 | -3.3 | 2.1 |
| | 100 | -17.6 | 8.0 | -3.9 | -18.8 | -8.1 | 13.8 | -10.1 | -2.3 | -9.8 | -12.4 | -8.6 | 4.8 | -3.6 | 5.8 | 9.1 | -27.7 | -4.1 | 17.4 |
| | 3 | -12.9 | -1.6 | 15.3 | -37.0 | -9.0 | 24.1 | -27.3 | 7.2 | -35.8 | -19.6 | -18.9 | 22.9 | 0.0 | 10.7 | -18.1 | 45.8 | 9.6 | 24.5 |
| Liver | 10,000 | -0.9 | -3.3 | -5.6 | -2.1 | -3.0 | 2.1 | 3.7 | 8.0 | 2.9 | 4.0 | 4.7 | 2.2 | -0.6 | -2.1 | 0.5 | 1.4 | -0.2 | 1.5 |
| | 1,000 | 2.0 | 0.5 | 3.0 | 0.8 | 1.6 | 1.1 | 12.4 | 8.2 | 6.5 | 6.9 | 8.5 | 2.5 | 2.5 | 2.5 | 1.5 | -0.9 | 1.4 | 1.6 |
| | 100 | 5.2 | -2.0 | 1.5 | 3.8 | 2.1 | 3.1 | 4.7 | 6.7 | -1.0 | 1.4 | 3.0 | 3.3 | -3.8 | 1.1 | -3.1 | -2.5 | -2.1 | 2.2 |
| | 3 | -11.3 | -11.2 | -9.2 | 1.6 | -7.5 | 6.6 | 43.5 | -9.1 | 12.3 | 19.3 | 16.5 | 18.6 | -2.7 | 16.7 | -0.2 | -28.0 | -3.5 | 19.1 |
| Heart | 10,000 | -21.4 | -10.5 | -17.1 | -11.8 | -15.2 | 5.9 | 2.9 | 2.2 | 2.3 | 1.7 | 2.3 | 0.5 | -3.9 | -3.9 | -2.5 | -3.7 | -3.5 | 0.7 |
| | 1,000 | -8.4 | -18.3 | -25.7 | -15.8 | -17.1 | 8.6 | 5.8 | 6.5 | 5.2 | 5.9 | 5.8 | 0.5 | -0.6 | 1.3 | 0.1 | -1.5 | -0.2 | 1.2 |
| | 100 | -13.7 | -15.8 | -15.0 | -21.9 | -16.6 | 4.3 | 4.5 | 6.2 | 3.7 | 8.5 | 5.7 | 2.0 | -3.3 | -2.0 | -1.9 | -12.6 | -4.9 | 5.4 |
| | 3 | 1.0 | -16.2 | 0.0 | -6.0 | -5.3 | 8.3 | 32.3 | -5.1 | 27.2 | 22.9 | 19.3 | 14.0 | 15.7 | 22.2 | 5.7 | 4.6 | 12.1 | 7.5 |

**[Table 2-2]**

| | | 1^{st} assay | | | | | | 2^{nd} assay | | | | | | 3^{rd} assay | | | | | |
|---|---|---|---|---|---|---|---|---|---|---|---|---|---|---|---|---|---|---|---|
| | | Relative error(%) | | | | | | Relative error(%) | | | | | | Relative error(%) | | | | | |
| | Nominal concentration (cells/50 uL blood or 20mg tissue or 10mg tissue) | No.1 | No.2 | No.3 | No.4 | Mean | Coefficient of variation (%) | No.1 | No.2 | No.3 | No.4 | Mean | Coefficient of variation (%) | No.1 | No.2 | No.3 | No.4 | Mean | Coefficient of variation (%) |
| Kidney | 10,000 | -4.5 | -7.5 | -4.5 | -5.9 | -5.6 | 1.5 | 3.6 | 3.3 | 1.7 | 1.5 | 2.5 | 1.1 | -7.3 | -5.4 | -3.3 | -4.0 | -5.0 | 1.8 |
| | 1,000 | 0.1 | -1.3 | 0.1 | -3.2 | -1.1 | 1.6 | 10.0 | 5.0 | 9.4 | 4.4 | 7.2 | 2.7 | -0.1 | 0.2 | 0.9 | -0.5 | 0.1 | 0.6 |
| | 100 | -6.7 | -2.5 | -3.3 | -6.6 | -4.8 | 2.3 | 8.0 | -4.1 | -1.1 | -4.7 | -0.5 | 5.9 | -3.3 | -1.0 | -1.8 | -7.3 | -3.4 | 2.9 |
| | 3 | -13.5 | -24.3 | 3.3 | -10.3 | -11.2 | 12.8 | 43.6 | -24.5 | 15.1 | -17.6 | 4.2 | 30.2 | 4.0 | -27.3 | -10.3 | 19.9 | -3.4 | 20.9 |
| Spleen | 10,000 | -13.0 | -13.4 | -15.1 | -14.7 | -14.0 | 1.2 | -4.4 | -0.2 | -0.4 | -0.2 | -1.3 | 1.0 | -6.8 | -11.2 | -11.7 | -6.2 | -9.0 | 2.3 |
| | 1,000 | -7.9 | -7.2 | -5.9 | 9.7 | -2.8 | 8.6 | 1.3 | 3.0 | -0.8 | -0.2 | 0.8 | 1.7 | -6.3 | -5.5 | -6.6 | -7.8 | -6.5 | 0.8 |
| | 100 | -9.8 | -9.8 | -12.2 | -10.4 | -10.5 | 1.3 | 0.0 | 1.7 | -4.9 | 11.1 | 2.0 | 4.7 | -6.7 | -5.6 | -11.0 | -8.1 | -7.8 | 2.6 |
| | 3 | -5.4 | -6.3 | -9.0 | -10.8 | -7.9 | 2.7 | 96.0 | 3.1 | 19.5 | 7.6 | 31.5 | 12.6 | -9.3 | -11.4 | 5.6 | 24.1 | 2.2 | 5.0 |
| Lung | 10,000 | -9.0 | -10.6 | -8.6 | -13.6 | -10.5 | 2.5 | 1.8 | 1.4 | 1.8 | 3.6 | 2.2 | 2.1 | -3.3 | -8.1 | -7.4 | -6.9 | -6.4 | 3.2 |
| | 1,000 | -5.8 | -9.4 | -6.8 | -5.4 | -6.9 | 1.9 | 5.4 | 7.3 | 7.1 | 3.3 | 5.8 | 1.7 | -3.6 | -4.6 | -3.4 | -2.7 | -3.6 | 1.1 |
| | 100 | -8.0 | -3.9 | -5.4 | -8.3 | -6.4 | 2.3 | 1.4 | 4.5 | 10.2 | -1.2 | 3.7 | 6.6 | -8.1 | -7.1 | -2.7 | -7.1 | -6.2 | 2.6 |
| | 3 | -7.7 | -2.9 | -2.8 | -30.6 | -11.0 | 14.9 | 1.3 | 14.4 | 36.9 | 23.3 | 19.0 | 33.1 | -3.0 | -4.5 | -9.7 | -13.0 | -7.5 | 16.1 |
| Testis | 10,000 | -6.5 | -9.7 | -8.4 | -11.7 | -9.1 | 2.4 | -3.8 | -0.3 | 4.2 | 4.7 | 1.2 | 4.0 | -0.8 | -1.9 | -4.4 | -1.4 | -2.1 | 1.6 |
| | 1,000 | -3.5 | -4.2 | -5.4 | -5.8 | -4.7 | 1.1 | 6.0 | 7.4 | 5.3 | 7.3 | 6.5 | 1.0 | 0.5 | 1.8 | 1.0 | 2.1 | 1.3 | 0.7 |
| | 100 | -3.3 | -6.6 | -6.4 | -4.5 | -5.2 | 1.6 | 9.4 | 0.7 | 6.4 | -1.7 | 3.7 | 4.9 | -7.1 | -2.1 | -7.3 | -4.1 | -5.1 | 2.6 |
| | 3 | 6.2 | 17.5 | 16.7 | -2.1 | 9.6 | 8.5 | 24.9 | -18.3 | 17.7 | 16.9 | 10.3 | 17.6 | 27.1 | 14.5 | -0.7 | 13.7 | 13.7 | 10.0 |

### Test Example 3 Study of phylogenetic difference/amplification of human cell-derived DNA in NSG and NOG mouse samples

A DNA/RNA shield solution (200 µL, ZYMO RESEARCH) in which 20 mg of liver of C.B-17 SCID mouse was dissolved was used as a specimen, and calibration curve samples (n = 2) obtained by adding, to the specimen, DNA/RNA shield solutions (70 µE) in which human cells (peripheral blood T cells, Cryo-T8: Human CD8⁺ Negatively Selected (5-8M cells), Precision Bioservices) corresponding to 3, 100, 1,000, 3,000, 5,000, and 10,000 cells were dissolved and a blank sample obtained by adding a DNA/RNA shield solution (70 µL) to which human cells were not added were prepared. QC samples (n = 4) corresponding to 100 cells were prepared by the same method as described above using the liver, heart, kidney, spleen, lung, testis, and blood of NOD.Cg-Prkdc^{scid}I12rg^{tm1Wjl}/SzJ mouse (NSG) and NOD.Cg-Prkdc^{scid}Il2rg^{tm1Sug}/ShiJic mouse (NOG) as specimens. For DNA recovery rate normalization, Dog Genomic DNA, Male (Zyagen) was added as an external standard gene to all samples. Genomic DNA was extracted from each of these samples using a commercially available DNA extraction reagent (MagMAX DNA Multi-Sample Ultra 2.0 Kit, Applied Biosystems) and Kingfisher Flex (Thermo Fisher Scientific Inc.), and then ddPCR quantification was performed.

ddPCR was quantified by duplex measurement using ddPCR Multiplex Supermix (BIO-RAD), DTT (BIO-RAD), restriction enzyme ScaI (Takara Bio Inc.), the probe of SEQ ID NO: 1 (5 pmol/well), the forward primer of SEQ ID NO: 2 (54 pmol/well) and the reverse primer of SEQ ID NO: 3 (54 pmol/well), the probe of SEQ ID NO: 4 (5 pmol/well), the forward primer of SEQ ID NO: 5 (10 pmol/well), and the reverse primer of SEQ ID NO: 6 (10 pmol/well). ddPCR was performed using QX200 Droplet Digital PCR System (BIO-RAD) at 95°C for 10 minutes/94°C for 30 seconds/(94°C for 30 seconds and 58°C for 1.5 minutes), which was regarded as one cycle, DNA was amplified at 45 cycles/98°C for 10 minutes, and quantification was performed.

A calibration curve was created from a value obtained by dividing the number of copies of the target gene by the number of copies of the external standard gene and the number of cells of the calibration curve sample. The results of calculating the number of cells of QC samples of NSG and NOG mice using the liver calibration curve of the C.B-17 SCID mouse are shown in Table 3. In any of the QC samples of the mouse, the mean value of the accuracy (RE) of the values calculated with respect to the number of added cells (Nominal), the accuracy (RE) of the QC sample of 50% or more in each matrix, and the accuracy (RE) and the precision (CV) of the QC sample of 2/3 or more of the total were within ±35%, which were favorable. By using this method, it was found that even samples of different strains can be quantified by using the same calibration curve.

**[Table 3]**

| | | NSG | | | | | | NOG | | | | | |
|---|---|---|---|---|---|---|---|---|---|---|---|---|---|
| | Nominal concentration (cells/50 µL blood or 20mg tissue or 10mg tissue) | No.1 | No.2 | No.3 | No.4 | Mean | Coefficient of variation (%) | No.1 | No.2 | No.3 | No.4 | Mean | Coefficient of variation (%) |
| Blood | 100 | -11.6 | -13.4 | -11.2 | -17.2 | -13.3 | 3.1 | -15.3 | -13.0 | -15.3 | -19.3 | -15.7 | 3.1 |
| Liver | 100 | 5.1 | 4.8 | 0.7 | -0.7 | 2.5 | 2.8 | 12.2 | 3.1 | -3.8 | 1.6 | 3.3 | 6.5 |
| Heart | 100 | 2.3 | 4.9 | 6.3 | 4.1 | 4.4 | 1.6 | 1.6 | 8.0 | 6.3 | 11.3 | 6.8 | 3.8 |
| Kidney | 100 | -2.1 | -14.0 | 4.9 | 0.2 | -2.7 | 8.3 | 1.6 | 2.5 | 6.1 | 4.9 | 3.8 | 2.0 |
| Spleen | 100 | 0.2 | -0.6 | -2.9 | -1.4 | -1.2 | 1.4 | -35.0 | 5.5 | 3.7 | 0.2 | -6.4 | 20.5 |
| Lung | 100 | 4.8 | 4.3 | 4.6 | -1.7 | 3.0 | 3.0 | -25.1 | -2.3 | -1.0 | 1.4 | -6.8 | 13.2 |
| Testis | 100 | 3.1 | 0.4 | 4.9 | 3.0 | 2.9 | 1.8 | -50.6 | 5.6 | 2.4 | 13.4 | -7.3 | 31.5 |

### Test Example 4 Cell administration test/amplification of human cell-derived DNA in C.B-17 SCID mouse sample

The C.B-17 SCID mouse was IV administered with human cells corresponding to 1,000,000 cells (peripheral blood T cells, Cryo-T8: Human CD8⁺ Negatively Selected (5-8M cells), Precision Bioservices), the liver, kidney, heart, lung, spleen, testis, and blood for 10 minutes, 1 hour, and 24 hours after administration were collected and used for DNA extraction as cell administration samples. A DNA/RNA shield solution (200 µL, ZYMO RESEARCH) in which 20 mg of liver of C.B-17 SCID mouse was dissolved was used as a specimen for a calibration curve, and calibration curve samples (n = 2) obtained by adding, to the specimen, DNA/RNA shield solutions (70 µE) in which human cells (peripheral blood T cells, Cryo-T8: Human CD8⁺ Negatively Selected (5-8M cells), Precision Bioservices) corresponding to 3, 100, 1,000, 3,000, 5,000, and 10,000 cells were dissolved and a blank sample obtained by adding a DNA/RNA shield solution (70 µL) to which human cells were not added were prepared. For the collected sample, a DNA/RNA shield solution (200 µL, ZYMO RESEARCH) in which liver, kidney, heart, lung, spleen, and testis were dissolved, and 50 µL of blood were used as specimens. Dog Genomic DNA, Male (Zyagen) was added as an external standard gene for DNA recovery rate normalization to all samples. Genomic DNA was extracted from each of these samples using a commercially available DNA extraction reagent (MagMAX DNA Multi-Sample Ultra 2.0 Kit, Applied Biosystems) and Kingfisher Flex (Thermo Fisher Scientific Inc.), and then ddPCR quantification was performed.

ddPCR was quantified by duplex measurement using ddPCR Multiplex Supermix (BIO-RAD), DTT (BIO-RAD), restriction enzyme ScaI (Takara Bio Inc.), the probe of SEQ ID NO: 1 (5 pmol/well), the forward primer of SEQ ID NO: 2 (54 pmol/well) and the reverse primer of SEQ ID NO: 3 (54 pmol/well), the probe of SEQ ID NO: 4 (5 pmol/well), the forward primer of SEQ ID NO: 5 (10 pmol/well), and the reverse primer of SEQ ID NO: 6 (10 pmol/well). ddPCR was performed using QX200 Droplet Digital PCR System (BIO-RAD) at 95°C for 10 minutes/94°C for 30 seconds/(94°C for 30 seconds and 58°C for 1.5 minutes), which was regarded as one cycle, DNA was amplified at 45 cycles/98°C for 10 minutes, and quantification was performed.

A calibration curve was created from a value obtained by dividing the number of copies of the target gene by the number of copies of the external standard gene and the number of cells of the calibration curve sample. Using this calibration curve, the number of cells in each tissue and blood sample of a mouse to which human cells (peripheral blood T cells, Cryo-T8: Human CD8⁺ Negatively Selected (5-8M cells), Precision Bioservices) were intravenously administered at 1,000,000 cells/animal could be calculated. The results are shown in Fig. 3.

### Test Example 5 Amplification of human cell-derived DNA in C.B-17 SCID mouse sample (REXO1L1)

Using the DNA extract extracted in Test Example 2, ddPCR quantification was performed using the REXO1L1 gene. Samples ranging from 10 to 10000 cells for the calibration curve and from 100 to 10000 cells for the QC sample were measured.

ddPCR was quantified by duplex measurement using ddPCR Multiplex Supermix (BIO-RAD), DTT (BIO-RAD), restriction enzyme ScaI (Takara Bio Inc.), the probe of SEQ ID NO: 7 (5 pmol/well), the forward primer of SEQ ID NO: 8 (54 pmol/well) and the reverse primer of SEQ ID NO: 9 (54 pmol/well), the probe of SEQ ID NO: 4 (5 pmol/well), the forward primer of SEQ ID NO: 5 (10 pmol/well), and the reverse primer of SEQ ID NO: 6 (10 pmol/well). ddPCR was performed using QXONE (BIO-RAD) at 95°C for 10 minutes/94°C for 30 seconds/(94°C for 30 seconds and 58°C for 1.5 minutes), which was regarded as one cycle, DNA was amplified at 45 cycles/98°C for 10 minutes, and quantification was performed.

A calibration curve was created from a value obtained by dividing the number of copies of the target gene by the number of copies of the external standard gene and the number of cells of each calibration curve sample. The results of calculating the number of cells of each QC sample using this calibration curve are shown in Table 4. The mean value of the accuracy (RE) of the values calculated with respect to the number of added cells (Nominal), the accuracy (RE) of the QC sample of 50% or more at each concentration, and the accuracy (RE) and the precision (CV) of the QC sample of 2/3 or more of the total were within ±35%, which were favorable.

**[Table 4]**

| | | | | |
|---|---|---|---|---|
| | | | | |

| | Nominal concentration (cells/50 µL blood or 20mg tissue or 10mg tissue) | Mean obserbed concentration (cells/50 µL blood or 20mg tissue or 10mg tissue) | Relative error (%) | Coefficient of variation (%) |
|---|---|---|---|---|
| Blood | 10,000 | 8,753 | -12.5 | 11.9 |
| | 1,000 | 764 | -23.6 | 2.7 |
| | 100 | 83 | -16.6 | 12.3 |
| Liver | 10,000 | 10,442 | 4.4 | 0.4 |
| | 1,000 | 1,053 | 5.3 | 2.7 |
| | 100 | 109 | 8.5 | 5.7 |
| Heart | 10,000 | 9,857 | -1.4 | 4.3 |
| | 1,000 | 930 | -7.0 | 6.6 |
| | 100 | 96 | -3.9 | 5.1 |
| Kidney | 10,000 | 10,375 | 3.7 | 1.5 |
| | 1,000 | 1,067 | 6.7 | 1.9 |
| | 100 | 109 | 9.0 | 4.8 |
| Spleen | 10,000 | 10,370 | 3.7 | 0.6 |
| | 1,000 | 1,076 | 7.6 | 9.2 |
| | 100 | 104 | 3.8 | 4.9 |
| Lung | 10,000 | 10,617 | 6.2 | 1.3 |
| | 1,000 | 1,050 | 5.0 | 1.8 |
| | 100 | 102 | 2.5 | 7.0 |
| Testis | 10,000 | 10,294 | 2.9 | 1.4 |
| | 1,000 | 1,009 | 0.9 | 1.1 |
| | 100 | 104 | 4.2 | 2.5 |

This application is based on Japanese Patent Application No. 2021-160065 filed on September 29, 2021 in Japan, the contents of which are encompassed in full herein. Sequence Listing

## Claims

1. A method for measuring a cell concentration in a sample, the method comprising the following steps of:
(1) measuring the number of copies of a specific gene and a gene of an external standard in a plurality of standard samples each having a known cell concentration by using digital PCR (dPCR);
(2) normalizing the measured value of the specific gene measured in the step (1) using the measured value of the external standard and creating a calibration curve on the basis of the normalized value and the known cell concentration;
(3) measuring the number of copies of the same specific gene and gene of the external standard in a sample having an unknown cell concentration by using dPCR; and
(4) normalizing the measured value of the specific gene measured in the step (3) using the measured value of the external standard and determining a cell concentration using the calibration curve created in the step (2) from the normalized value.

2. The method according to claim 1, wherein in the step (1), measurement of the number of copies of a specific gene and measurement of the number of copies of a gene of an external standard are simultaneously performed.

3. The method according to claim 1, wherein a matrix of each of the standard samples used for creating a calibration curve is different from a matrix of the sample used for measuring a cell concentration.

4. The method according to claim 1, wherein accuracy of the value of the cell concentration determined in the step (4) is within ±35%.

5. The method according to claim 1, wherein precision of the value of the cell concentration determined in the step (4) is within ±35%.

6. The method according to claim 1, wherein the specific gene is LINE-1 gene.

7. The method according to claim 1, wherein the specific gene is REXO1L1 gene.
